(19) Europäisches Patentamt
European Patent Office
Office européen des brevets

(11) EP 4 351 563 B1

(12) EUROPEAN PATENT SPECIFICATION

(45) Date of publication and mention of the grant of the patent:
**30.09.2026 Bulletin 2026/40**

(21) Application number: **22734239.1**

(22) Date of filing: **08.06.2022**

(51) International Patent Classification (IPC):
***A61K 31/337*** *(2006.01)* ***A61K 31/437*** *(2006.01)*
***A61K 31/444*** *(2006.01)* ***A61K 31/4709*** *(2006.01)*
***A61K 31/7068*** *(2006.01)* ***A61K 45/06*** *(2006.01)*
***A61P 35/00*** *(2006.01)*

(52) Cooperative Patent Classification (CPC):
(C-Sets available)
**A61K 31/437; A61K 31/337; A61K 31/444; A61K 31/4709; A61K 31/7068; A61K 45/06; A61P 35/00** (Cont.)

(86) International application number:
**PCT/EP2022/065562**

(87) International publication number:
**WO 2022/258693 (15.12.2022 Gazette 2022/50)**

# (54) AUTOTAXIN (ATX) INHIBITOR FOR THE TREATMENT OF PANCREATIC CANCER

AUTOTAXIN (ATX)-INHIBITOR ZUR BEHANDLUNG VON PANKREASKREBS

INHIBITEUR DE L'AUTOTAXINE (ATX) POUR LE TRAITEMENT DU CANCER DU PANCRÉAS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.06.2021 GB 202108245**

(43) Date of publication of application:
**17.04.2024 Bulletin 2024/16**

(73) Proprietor: **iOnctura BV**
**1083 HN Amsterdam (NL)**

(72) Inventors:
- **JOHNSON, Zoë**
  **1202 Genève (CH)**
- **DEKEN, Marcel**
  **1202 Genève (CH)**
- **LAHN, Michael**
  **1202 Genève (CH)**
- **MELISI, Davide**
  **37134 Verona (IT)**

(74) Representative: **Mewburn Ellis LLP**
**Aurora Building**
**Counterslip**
**Bristol BS1 6BX (GB)**

(56) References cited:
**WO-A1-2016/124939**

- **BURRIS H A ET AL: "Improvements in survival and clinical benefit with gemcitabine as first-line therapy for patients with advanced pancreas cancer: a randomized trial", vol. 15, no. 6, 1 June 1997 (1997-06-01), pages 2403 - 2413, XP008104764, ISSN: 0732-183X, Retrieved from the Internet <URL:http://jco.ascopubs.org/cgi/content/abstract/15/6/2403> [retrieved on 20220914]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/337, A61K 2300/00;**
**A61K 31/437, A61K 2300/00;**
**A61K 31/444, A61K 2300/00;**
**A61K 31/4709, A61K 2300/00;**
**A61K 31/7068, A61K 2300/00**

## Description

This application claims priority from GB2108245.8 filed 9 June 2021.

### *Field of the Invention*

The present invention relates to a compound or pharmaceutically acceptable salt thereof for use in a method of treatment of pancreatic cancer, and to combination methods including said compound.

### *Background*

Pancreatic cancer is a malignant tumour of the pancreas. Pancreatic cancer has been called a 'silent' disease because early pancreatic cancer usually does not cause symptoms. It is therefore difficult to detect in its early stages.

Pancreatic cancer is one of the deadliest types of cancer with a very poor 5-year survival rate of only 7%. A mere 25% of pancreatic cancer patients are surgical candidates at the time of diagnosis, and of those who receive surgical resection, only about 20% live longer than 5 years. Chemotherapy with gemcitabine is a standard treatment with a 5-10% response rate and average median overall survival of 6 months (Burris et al. 1997).

Pancreatic ductal adenocarcinoma (PDAC), the most prevalent form of pancreatic cancer, is a growing health problem with increasing mortality worldwide, exerting a huge economic burden on our healthcare systems and significantly impacting the quality of life of patients. It is predicted that PDAC will become the second leading cause of cancer death in some regions. The incidence of pancreatic cancer is increasing in the Western world and a better understanding of the risk factors and symptoms associated with this disease is needed to inform both health professionals and the general population of potential preventive and/or early detection measures. There is currently a lack of therapeutic approaches for early-stage detection which would increase patient survival.

Pancreatic cancer progression typically features a dramatic desmoplastic reaction, including fibroblasts, immune cells, and a dense extracellular matrix. The transforming growth factor-$\beta$ (TGF-$\beta$) pathway is one of the signalling systems that has been identified as a major contributor to the pathogenesis of the disease (Truty and Urrutia, 2007). Because of the highly fibrotic tumour microenvironment, conventional chemotherapy and radiotherapy have only moderate anti-tumour activity in pancreatic tumours. Similarly, immune therapies, which are highly effective in other cancer types, such as $\alpha$-PD-1 therapy, have shown to be ineffective in pancreatic cancer. Therefore, new treatments for pancreatic cancer are desperately needed.

Increasing evidence now supports a physiological role for lysophosphatidic acid (LPA) in regulating pancreatic cancer initiation, progression and metastasis (Chen et al. 2021). LPA is a bioactive phospholipid that engages at least six receptors, LPAR1-6, which are each coupled to a distinct G-protein that participates in various cellular activities such as cell migration, proliferation, and differentiation. LPA is present in various biological fluids, and its levels in plasma are well-characterized in terms of its role in blood coagulation.

LPA is generated from lysophosphatidylcholine (LPC) by the extracellular lysoPLD autotaxin (ATX), also referred to as ectonucleotide pyrophosphatase / phosphodiesterase 2 (ENPP2). Increased ATX expression has been reported in multiple cancers including pancreatic cancer. Overexpression of both ATX and LPA in pancreatic tissues has been reported for pancreatic cancer patients and thus the ATX-LPA axis may represent a potential target in pancreatic cancer.

There is increasing demand for targeted therapy in pancreatic cancer treatment. Targeted therapy is directed to specific receptors or enzymes that are present in the tumour and does not harm the healthy tissue unlike the traditional therapeutic methods like chemotherapy.

WO2016124939 describes various ATX inhibitor compounds, including N-[(S)-1-(4-chloro-phenyl)-ethyl]-3-[3-( 4-trifluoromethoxy-benzyl)-3H-imidazo[4,5-b]pyridin-2-yl]-propionamide, and their use in the treatment of proliferative disorders in which ATX activity is implicated.

### *Summary of the Invention*

The present invention is directed to a compound for use in the treatment of pancreatic cancer. The compound is an ATX inhibitor. The inventors recognised that ATX inhibitors may be useful in targeted therapy for the treatment of pancreatic cancer.

In a first aspect, the invention provides a compound of Formula I:

Formula I

or a pharmaceutically acceptable salt thereof for use in the treatment of pancreatic cancer in a patient. The compound of Formula I may be referred to herein as "Compound 1".

The term pancreatic cancer includes any exocrine or neuroendocrine pancreatic cancer type. In some cases, wherein the pancreatic cancer is pancreatic ductal adenocarcinoma (PDAC). PDAC is the most prevalent neoplastic disease of the pancreas, accounting for more than 90% of all pancreatic malignancies.

Suitably, Compound 1 is administered in a pharmaceutical composition comprising compound 1 or a pharmaceutically acceptable salt thereof and a pharmaceutically acceptable diluent, carrier or excipient. Preferably, but not necessarily, the pharmaceutical composition is suitable for oral administration.

In some cases, the treatment is a combination therapy and comprises one or more additional chemotherapeutic agents and/or a TGF- β pathway inhibitor. The inventors have observed that the administration of Compound 1 with an approved chemotherapeutic agent both improved tumour growth inhibition and reduced adverse effects observed for the chemotherapeutic agent alone.

For example, the method may comprise administration of a therapeutically effective amount of an additional chemotherapeutic agent, optionally two additional chemotherapeutic agents. Suitable chemotherapeutics agents include gemcitabine and nab-paclitaxel. Accordingly, methods of the present invention may comprise administration of gemcitabine and/or nab-paclitaxel.

The inventors have observed improved treatment outcomes, in particular with respect to patient survival, when a TGF-β pathway inhibitor is administered. Suitable TGF-β pathway inhibitors may include galunisertib, vactosertib, LY3200882 and AVID200.

Accordingly, in some cases the method comprises administration of a therapeutically effective amount of a TGF-β pathway inhibitor, for example, galunisertib. In some cases, the method comprises a triple therapy of Compound 1, or a pharmaceutically acceptable salt thereof, a TGF- β pathway inhibitor and an additional chemotherapeutic agent, optionally two additional chemotherapeutic agents. In some cases, the triple therapy is Compound 1, or a pharmaceutically acceptable salt thereof, galunisertib, and gemcitabine.

***Summary of the Figures***

Embodiments and experiments illustrating the principles of the invention will now be discussed with reference to the accompanying figures in which:

**Figure 1** shows the expression of ATX in pancreatic cancer samples compared to healthy tissue

**Figure 2** shows the anti-tumour growth activity of Compound 1 in the mPA6115-luc (MuPrime) mouse model

**Figure 3** shows the activity of Compound 1 in the Panc-1 mouse xenograft model

**Figure 4** shows the activity of Compound 1 plus gemcitabine in the orthotopic Panc-1 mouse model

**Figure 5** shows the activity of Compound 1 plus gemcitabine and/or galunisertib in the RC416 orthotopic mouse model.

***Detailed Description of the Invention***

Aspects and embodiments of the present invention will now be discussed. Further aspects and embodiments will

be apparent to those skilled in the art.

*Compound 1*

WO2016124939 describes various ATX inhibitor compounds and their use in the treatment of proliferative disorders in which ATX activity is implicated, including Compound 1.

Compound 1 is example 40 in WO2016124939. WO2016124939 describes over 200 examples. Compound 1's structure is according to Formula I.

Formula I.

Its IUPAC name is *N*-[(S)-1-(4-chloro-phenyl)-ethyl]-3-[3-( 4-trifluoromethoxy-benzyl)-3*H*-imidazo[4,5-b]pyridin-2-yl]-propionamide. Its synthesis and characterisation are described in WO2016124939 at pages 77 and 82, respectively.

Compound 1 may be provided and administered as the free base or as a pharmaceutically acceptable salt. In some cases, Compound 1 is provided and administered as the free base.

*Formulation*

Suitability, Compound 1 is provided in a pharmaceutical composition formulated for oral administration. The pharmaceutical composition may be provided in a capsule or may be provided in a tablet. In some cases, it is provided in a tablet. In other cases, it is provided in a capsule, for example, as a powdered or granulated composition or a liquid composition within a hard- or soft-shell capsule, for example, a hydroxymethyl cellulose (HPMC) capsule. In other words, an oral dosage form is preferred.

The formulation suitably comprises one or more pharmaceutically acceptable fillers, disintegrants, glidants, and/or lubricants.

*Treatment of pancreatic cancer using Compound 1*

Compound 1 is an ATX inhibitor. ATX is an attractive target for the treatment of pancreatic cancer because it acts extracellularly and stimulates cancer growth, survival and metastasis at multiple levels.

It is recognized in the art that the LPA-ATX pathway is frequently activated in pancreatic cancer. The methods of the present invention may therefore relate to treatment of pancreatic cancer characterised by upregulation of the ATX-LPA pathway. The methods of the present invention may therefore relate to treatment of pancreatic cancer by modulation of ATX-LPA pathway in a patient.

The pancreatic cancer may be any exocrine or neuroendocrine pancreatic cancer type. Accordingly, the methods of the present invention are directed to the treatment of pancreatic cancer, such as but not limited to pancreatic ductal adenocarcinoma (PDAC) and pancreatic neuroendocrine tumours (PanNETs or PNETs). In some cases, the pancreatic cancer is pancreatic ductal adenocarcinoma. In some cases, the pancreatic cancer is pancreatic neuroendocrine tumours.

*Methods of the invention*

As described in more detail below, the present inventors have surprisingly found that Compound 1 shows robust anti-tumour activity in preclinical models of pancreatic cancer and is well tolerated. Thus, an ATX inhibitor with anti-tumour activity and favourable safety characteristics in pancreatic cancer can be provided.

Additionally, the present inventors surprisingly found that Compound 1 increases the anti-tumour activity of

standard of care chemotherapies such as gemcitabine. Thus, an ATX inhibitor that can increase the efficacy of chemotherapy in pancreatic cancer can be provided.

Accordingly, in some cases the methods of the invention are directed to combination therapy, the combination therapy comprising treatment of a patient with Compound 1 or a pharmaceutically acceptable salt thereof and an additional chemotherapeutic agent, for example gemcitabine (Gemzar®) or nab-paclitaxel (Abraxane®).

It will be appreciated that Compound 1 and the additional chemotherapeutic agent will suitably, although not necessarily, be given at different times and/or on different schedules and may be formulated for administration by different routes. For example, Compound 1 or a pharmaceutically acceptable salt thereof may be given as an oral dose, for example, a daily oral dose, while the additional chemotherapeutic agent may be given as infusion. For example both gemcitabine and nab-paclitaxel may be given in a 28 day cycle on days 1, 8, and 15.

In some cases, the combination therapy comprises treating the patient with Compound 1 or a pharmaceutically acceptable salt thereof and gemcitabine and nab-paclitaxel.

Furthermore, the inventors have found that a triple combination of Compound 1, galunisertib (a TGF-β pathway inhibitor) and chemotherapy represents a further improvement on outcome in a preclinical pancreatic cancer model. In some cases, the methods include administration of a TGF-β pathway inhibitor. Suitable TGF-β pathway inhibitors may include galunisertib, vactosertib, LY3200882 and AVID200.

Accordingly, in some cases the methods of the invention are directed to combination therapy, the combination therapy comprising treatment of a patient with Compound 1 or a pharmaceutically acceptable salt thereof, a TGF-β pathway inhibitor such as galunisertib (LY2157299 monohydrate, Eli Lilley), and an additional chemotherapeutic agent, for example gemcitabine and/or nab-paclitaxel (Abraxane®).

It will be appreciated that Compound 1 and the TGF-β pathway inhibitor agent will suitably, although not necessarily, be given at different times and/or on different schedules and may be formulated for administration by different routes.

Therefore, the inventors have found that, surprisingly, pancreatic cancer can be treated with Compound 1 with or without chemotherapy and with or without TGF-β pathway inhibitors and this treatment is well tolerated. This provides new monotherapy and combination therapy options for the treatment of pancreatic cancer.

The dose of Compound 1 may be provided once daily (QD), preferably twice daily (BID), preferably but not necessarily administered orally. Other methods of administration may be used. A suitable daily dose may be between 5 mg and 2 g, for example between 10 mg and 1g. In some cases, where Compound 1 is administered in a combination therapy, administration of Compound 1 continues during pauses in administration of other agents (for example, during days 21-28 of 28 day chemotherapeutic cycles).

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof. While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### *Examples*

*EXAMPLE 1*

**ATX (ENPP2) expression in pancreatic cancer**

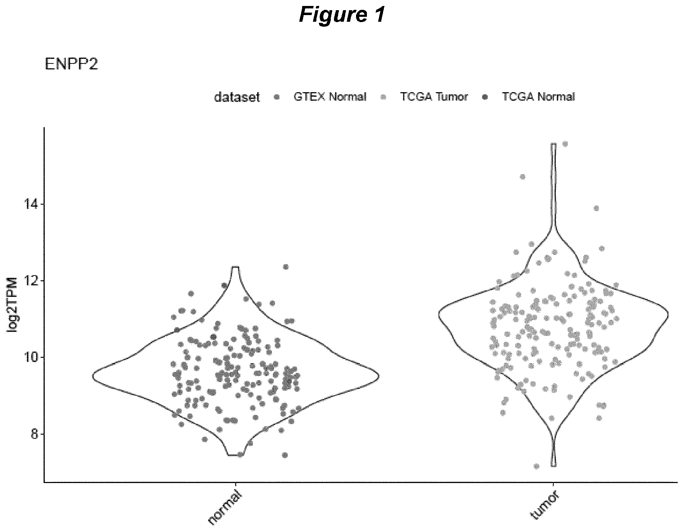

ENPP2 (the gene encoding autotaxin) expression in human pancreatic cancer samples was compared to normal pancreatic tissue using TCGA (cancer tissues) and GTEX (normal tissues) data. ENPP2 expression in tumor samples was found to be 1.85 times higher than in normal tissue (see **Figure 1** and **Table 1).**

**Table 1**

| | GTEX | TCGA |
|---|---|---|
| Metastatic | 0 | 1 |
| Normal Tissue | 167 | 0 |
| Primary Tumor | 0 | 178 |
| Solid Tissue Normal | 0 | 4 |

*EXAMPLE 2*

**Activity of Compound 1 in the orthotopic mPA6115-luc mouse model**

The therapeutic efficacy of Compound 1 was evaluated in the orthotopic mPA6115-luc mouse model of pancreatic cancer. This model is constituted by the implantation of mPA6115-luc cells originating from a spontaneous developed donor tumour in KPC mice into the pancreas of female wildtype C57BL/6 recipient mice. The pathology and tumour microenvironment in this model closely resemble human pancreatic cancer and is characterized by limited immune cell infiltration.

Each mouse was inoculated into the subcapsular region of the pancreas with mPA6115-luc tumour cells (1 x $10^6$) in 50uL PBS with Matrigel (1:1). The date of tumour cell inoculation was denoted as day 0.

The randomization started 4 days after tumour cell inoculation based on the total flux (p/s, minimum flux >1E$^6$). 10 mice per group were assigned to treatment by BID oral gavage with vehicle (1% methylcellulose) or Compound 1 (10 mg/kg in 1% methylcellulose). Tumour growth and metastasis were imaged twice per week by bioluminescent imaging.

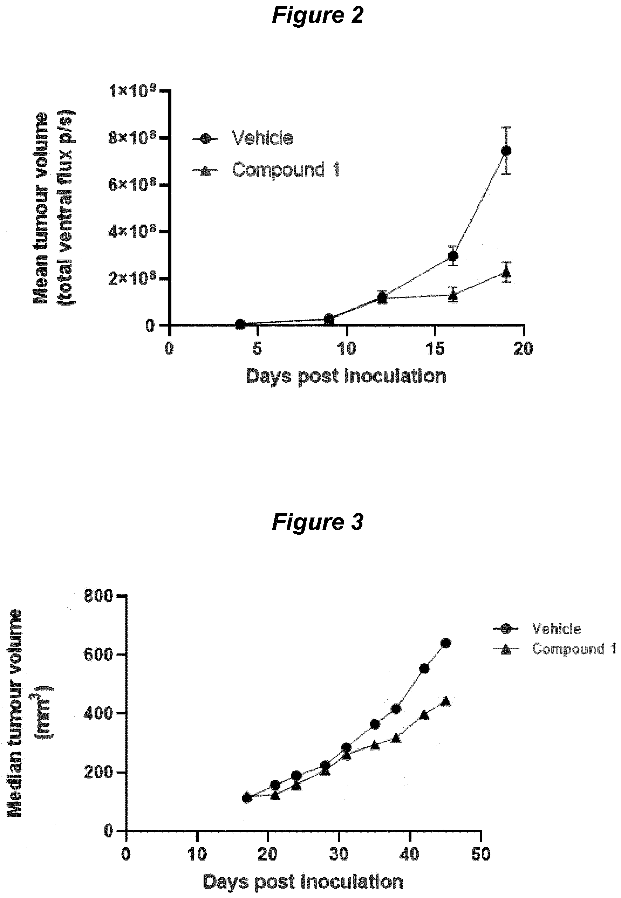

As can be seen in **Figure 2,** treatment of mPA6115-luc tumour-bearing mice with Compound 1 demonstrated significantly reduced tumour outgrowth based on total bioluminescence.

*EXAMPLE 3*

**Activity of Compound 1 in the xenograft PANC-1 mouse model**

The therapeutic efficacy of Compound 1 was evaluated in the subcutaneous PANC-1 mouse model of pancreatic cancer.

BALB/c Nude mice were inoculated subcutaneously in the right front flank region with PANC-1 tumour cells (5 x $10^6$) in 0.1 ml of PBS. The date of tumour cell inoculation was denoted as day 0. The randomization started when the mean tumour size >100 mm$^3$. 10 mice were enrolled per study group. Tumour growth and body weights were measured twice per week.

As seen in **Figure 3,** treatment of PANC-1 tumour-bearing mice with Compound 1 demonstrated reduced tumour outgrowth.

*EXAMPLE 4*

**Activity of Compound 1 plus gemcitabine in the orthotopic PANC-1 mouse model**

The therapeutic efficacy of Compound 1 with or without gemcitabine was evaluated in the orthotopic PANC-1 mouse model of pancreatic cancer. This model is constituted by the implantation of PANC-1 tumor cells into the pancreas of cells in BALB/C nude mice.

Each mouse was inoculated into the subcapsular region of the pancreas with PANC-1 tumour cells (3 x $10^6$) in 50uL PBS with Matrigel (1:1). The date of tumour cell inoculation was denoted as day 0. Randomization started 10 days after tumour cell inoculation based on body weight. 10 mice per group were assigned to treatment by oral gavage with vehicle (1% methylcellulose, BID), gemcitabine (25 mg/kg, Q4D), Compound 1 (10 mg/kg, BID), or Compound 1 (10 mg/kg, BID) and gemcitabine (25 mg/kg, Q4D). Tumor sizes were measured by weight after termination at day 42.

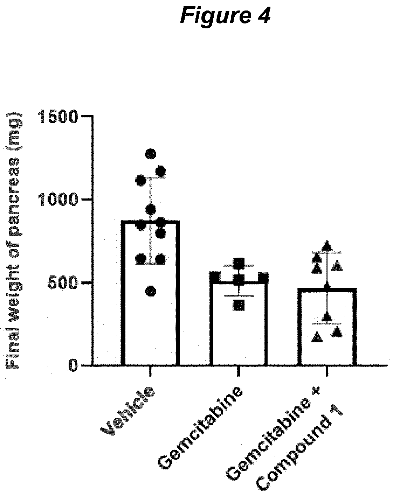

As depicted in **Figure** 4, treatment of the PANC-1 tumour-bearing mice with gemcitabine alone demonstrated tumour growth inhibition of 41% but the treatment was poorly tolerated and 5 mice died or were sacrificed for reaching humane endpoints. Treatment with Compound 1 alone resulted in a modest tumour growth inhibition of 5%, however the combination of Compound 1 and gemcitabine did not only result in an improved tumour growth inhibition of 47%, it also reduced the mortality of gemcitabine treatment to only two mice. See also **Table 2.**

| Treatment Description | Number of Mice on Day 42 | Pancreas Size (mg) on Day 42 |
|---|---|---|
| **Vehicle** | 10 | 874±259 |
| **Gemcitabine, 25 mg/kg** | 5 | 549±44 |
| **Compound 1, 10 mg/kg + Gemcitabine, 25 mg/kg** | 8 | 468±213 |

**Table 2**

<u>*EXAMPLE 5*</u>

**Activity of Compound 1 plus gemcitabine and/or galunisertib in the orthotopic RC416 mouse model**

The therapeutic efficacy of Compound 1 with or without gemcitabine and/or galunisertib was evaluated in the orthotopic RC416 mouse model of pancreatic cancer. This model is constituted by the implantation of RC416 cells originating from a spontaneous developed donor tumour in KPC mice into the pancreas of female wildtype C57BL/6 recipient mice. The pathology and tumour microenvironment in this model closely resemble human pancreatic cancer and is characterized by high circulating ATX and TGF-β.

Each mouse was inoculated into the subcapsular region of the pancreas with RC416 tumour cells. The day of tumour cell inoculation was denoted as day 0. Randomization started 7 days after tumor cell inoculation, based on body weight. 5 mice per group were assigned to treatment , with vehicle (1% methylcellulose, BID p.o.), Compound 1 (10 mg/kg, BID p.o.), galunisertib (50 mg/kg, BID p.o.), gemcitabine (75 mg/kg, QW i.p.), or the combinations of Compound 1 plus gemcitabine, Compound 1 plus galunisertib and gemcitabine, and galunisertib plus gemcitabine, Mice were treated for a maximum of 28 days and anti-tumour activity was measured by survival.

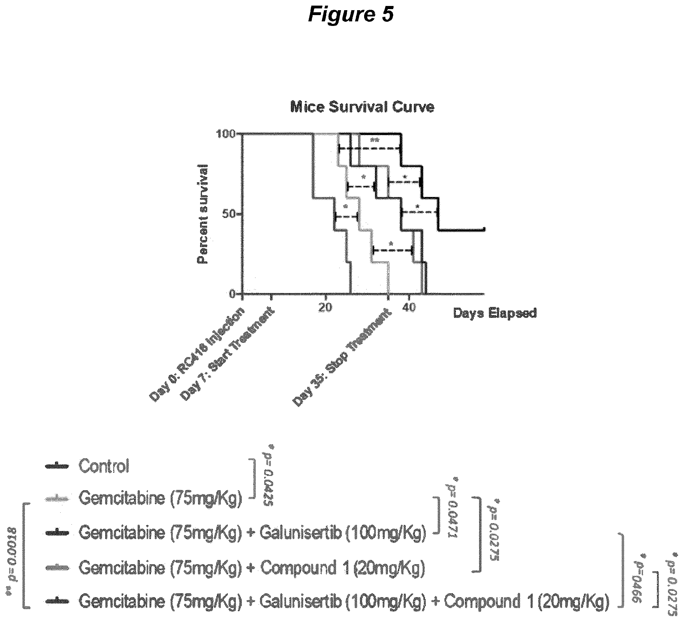

The results depicted in **Figure 5,** show that treatment with gemcitabine alone resulted in limited survival benefit. Surprisingly however, the combinations of gemcitabine plus Compound 1 or galunisertib resulted in almost doubling of the survival. Even more surprising, the combination of all three treatments further enhanced the overall survival with 2 mice surviving for over 65 days.

***References***

A number of publications are cited above in order to more fully describe and disclose the invention and the state of the art to which the invention pertains. Full citations for these references are provided below.

WO2016124939

Burris, H. A. et al. Improvements in survival and clinical benefit with gemcitabine as first-line therapy for patients with advanced pancreas cancer: a randomized trial. J Clin Oncol 15, 2403-2413 (1997).

Chen, J., Li, H., Xu, W. & Guo, X. Evaluation of serum ATX and LPA as potential diagnostic biomarkers in patients with pancreatic cancer. Bmc Gastroenterol 21, 58 (2021).

Truty, M. J. & Urrutia, R. Basics of TGF-β and Pancreatic Cancer. Pancreatology 7, 423-435 (2007).

## Claims

**1.** A compound of Formula I:

Formula I

or a pharmaceutically acceptable salt thereof for use in a method of treatment of pancreatic cancer in a patient.

**2.** The compound or salt for use according to claim 1, wherein the pancreatic cancer is pancreatic ductal adenocarcinoma (PDAC).

**3.** The compound or salt for use according to claim 1 or claim 2, wherein the compound of Formula I is administered in a pharmaceutical composition comprising said compound or a pharmaceutically acceptable salt thereof and a pharmaceutically acceptable diluent, carrier or excipient; wherein the pharmaceutical composition is suitable for oral administration.

**4.** The compound or salt for use according to any preceding claim, wherein the method comprises administration of a therapeutically effective amount of an additional chemotherapeutic agent.

**5.** The compound or salt for use according to claim 4, wherein the additional chemotherapeutic agent is gemcitabine.

**6.** The compound or salt for use according to claim 4, wherein the additional chemotherapeutic agent is nab-paclitaxel.

**7.** The compound or salt for use according to claim 4, wherein two additional chemotherapeutic agents are used.

**8.** The compound or salt for use according to claim 7, wherein the two additional chemotherapeutic agents are gemcitabine and nab-paclitaxel.

**9.** The compound or salt for use according to any preceding claim, wherein the method comprises administration of a therapeutically effective amount of a TGF-β pathway inhibitor.

**10.** The compound or salt for use according to claim 9, wherein the TGF-β pathway inhibitor is selected from galunisertib, vactosertib, LY3200882 and AVID200.

**11.** The compound or salt for use according to claim 9, wherein the TGF-β pathway inhibitor is galunisertib.

## Patentansprüche

**1.** Verbindung der Formel I:

Formel I

oder ein pharmazeutisch annehmbares Salz davon zur Behandlung von Bauchspeicheldrüsenkrebs bei einem Patienten.

2. Verbindung oder Salz zur Verwendung nach Anspruch 1, wobei der Bauchspeicheldrüsenkrebs ein duktales Adenokarzinom des Pankreas (PDAC) ist.

3. Verbindung oder Salz zur Verwendung nach Anspruch 1 oder Anspruch 2, wobei die Verbindung der Formel I in einer pharmazeutischen Zusammensetzung verabreicht wird, welche die Verbindung oder ein pharmazeutisch annehmbares Salz davon und einen pharmazeutisch annehmbaren Verdünner, Träger oder Hilfsstoff umfasst; wobei die pharmazeutische Zusammensetzung zur oralen Verabreichung geeignet ist.

4. Verbindung oder Salz zur Verwendung nach einem der vorangegangenen Ansprüche, wobei das Verfahren die Verabreichung einer therapeutisch wirksamen Menge eines weiteren Chemotherapeutikums umfasst.

5. Verbindung oder Salz zur Verwendung nach Anspruch 4, wobei das weitere Chemotherapeutikum Gemcitabin ist.

6. Verbindung oder Salz zur Verwendung nach Anspruch 4, wobei das weitere Chemotherapeutikum Nab-Paclitaxel ist.

7. Verbindung oder Salz zur Verwendung nach Anspruch 4, wobei zwei weitere Chemotherapeutika verwendet werden.

8. Verbindung oder Salz zur Verwendung nach Anspruch 7, wobei die zwei weiteren Chemotherapeutika Gemcitabin und Nab-Paclitaxel sind.

9. Verbindung oder Salz nach einem der vorangegangenen Ansprüche, wobei das Verfahren die Verabreichung einer therapeutisch wirksamen Menge eines TGF-β-Signalweg-Inhibitors umfasst.

10. Verbindung oder Salz zur Verwendung nach Anspruch 9, wobei der TGF-β-Signalweg-Inhibitor aus Galunisertib, Vactosertib, LY3200882 und AVID200 ausgewählt ist.

11. Verbindung oder Salz zur Verwendung nach Anspruch 9, wobei der TGF-β-Signalweg-Inhibitor Galunisertib ist.

**Revendications**

1. Composé de formule I :

Formule I

ou sel pharmaceutiquement acceptable de celui-ci pour utilisation dans une méthode de traitement du cancer du pancréas chez un patient.

2. Composé ou sel pour utilisation selon la revendication 1, dans lequel le cancer du pancréas est un adénocarcinome canalaire pancréatique (PDAC).

3. Composé ou sel pour utilisation selon la revendication 1 ou la revendication 2, dans lequel le composé de formule I est administré dans une composition pharmaceutique comprenant ledit composé ou un sel pharmaceutiquement acceptable de celui-ci et un diluant, un support ou un excipient pharmaceutiquement acceptable ; dans lequel la composition pharmaceutique est appropriée pour une administration orale.

4. Composé ou sel pour utilisation selon une quelconque revendication précédente, dans lequel le procédé comprend l'administration d'une quantité thérapeutiquement efficace d'un agent chimiothérapeutique supplémentaire.

5. Composé ou sel pour utilisation selon la revendication 4, dans lequel l'agent chimiothérapeutique supplémentaire est la gemcitabine.

6. Composé ou sel pour utilisation selon la revendication 4, dans lequel l'agent chimiothérapeutique supplémentaire est le nab-paclitaxel.

7. Composé ou sel pour utilisation selon la revendication 4, dans lequel deux agents chimiothérapeutiques supplémentaires sont utilisés.

8. Composé ou sel pour utilisation selon la revendication 7, dans lequel les deux agents chimiothérapeutiques supplémentaires sont la gemcitabine et le nab-paclitaxel.

9. Composé ou sel pour utilisation selon une quelconque revendication précédente, dans lequel le procédé comprend l'administration d'une quantité thérapeutiquement efficace d'un inhibiteur de la voie du TGF-β.

10. Composé ou sel pour utilisation selon la revendication 9, dans lequel l'inhibiteur de la voie du TGF-β est choisi parmi le galunisertib, vactosertib, LY3200882 et AVID200.

11. Composé ou sel pour utilisation selon la revendication 9, dans lequel l'inhibiteur de la voie du TGF-β est le galunisertib.

*Figure 1*

ENPP2
dataset
GTEX Normal
TCGA Tumor
TCGA Normal
log2TPM
14
12
10
8
normal
tumor

*Figure 2*

Mean tumour volume
(total ventral flux p/s)
1×10⁹
8×10⁸
6×10⁸
4×10⁸
2×10⁸
0
0
5
10
15
20
Days post inoculation
Vehicle
Compound 1

*Figure 3*

Median tumour volume
(mm³)
800
600
400
200
0
0
10
20
30
40
50
Days post inoculation
Vehicle
Compound 1

*Figure 4*

Final weight of pancreas (mg)
1500
1000
500
0
Vehicle
Gemcitabine
Gemcitabine +
Compound 1

# *Figure 5*

Mice Survival Curve
Percent survival
100
50
0
20
40
Days Elapsed
**
*
Day 0: RC416 Injection
Day 7: Start Treatment
Day 35: Stop Treatment

Control
Gemcitabine (75mg/Kg)
Gemcitabine (75mg/Kg) + Galunisertib (100mg/Kg)
Gemcitabine (75mg/Kg) + Compound 1 (20mg/Kg)
Gemcitabine (75mg/Kg) + Galunisertib (100mg/Kg) + Compound 1 (20mg/Kg)
* p= 0.0425
* p= 0.0471
* p= 0.0275
** p= 0.0018
* p=0466
* p= 0.0275

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 2108245 A
- WO 2016124939 A


### Non-patent literature cited in the description


- **BURRIS, H. A. et al.** Improvements in survival and clinical benefit with gemcitabine as first-line therapy for patients with advanced pancreas cancer: a randomized trial. *J Clin Oncol*, 1997, vol. 15, 2403-2413
- **CHEN, J.** ; **LI, H.** ; **XU, W.** ; **GUO, X.** Evaluation of serum ATX and LPA as potential diagnostic biomarkers in patients with pancreatic cancer. *Bmc Gastroenterol*, 2021, vol. 21, 58
- **TRUTY, M. J.** ; **URRUTIA, R.** Basics of TGF-β and Pancreatic Cancer. *Pancreatology*, 2007, vol. 7, 423-435